# FASCICULE DE BREVET EUROPEEN

(11) **EP 1 289 975 B1**
(45) Date de publication et mention de la délivrance du brevet: **02.02.2005**
(21) Numéro de dépôt: 01928013.0
(22) Date de dépôt: 19.04.2001
(51) Int. Cl.: C07D 303/36, C07D 303/46, A01N 43/20, D06M 13/47

(54) **SEL DE TRIALKYLAMMONIUM EPOXYDE, PROCEDE DE SYNTHESE ET APPLICATIONS**
TRIALKYLAMMONIUM EPOXID SALZ, VERFAHREN ZU DESSEN HERSTELLUNG UND VERWENDUNGEN
EPOXIDIZED TRIALKYLAMMONIUM SALT, SYNTHESIS METHOD AND USES

(30) Priorité: 19.04.2000 FR 0005059
(43) Date de publication de la demande: 12.03.2003
(73) Titulaire: Institut Textile et Chimique de Lyon ( ITECH LYON), 69130 Ecully (FR)
(72) Inventeur: LEMAIRE, Marc, F-69100 Villeurbanne (FR); GOZZI, Christel, F-69007 Lyon (FR); BOULON, Gilbert, F-69005 Lyon (FR); DALPHINET, André, F-69250 Poleymieux-au-Mont-d'Or (FR)
(74) Mandataire: Guerre, Dominique
(86) Numéro de dépôt international: PCT/FR2001/001214
(87) Numéro de publication internationale: WO 2001/079191

(56) Documents cités:
- EP-A- 0 591 024
- WO-A-00/05281
- WO-A-83/00513

## Description

La présente invention concerne la modification permanente des propriétés, notamment vis à vis des microorganismes, de substrats, par greffage covalent de composés présentant les propriétés recherchées pour ces substrats.

Les propriétés recherchées pour leur intérêt dans le domaine médical, ou dans des secteurs industriels utilisateurs de cellulose sous forme de papier, emballage et textile par exemple, sont notamment les propriétés biocide et/ou bactériostatique de contact.

Pour tous les objets utilisables, par exemple en chambre stérile, ou au contraire en milieu contaminant, comme les éléments de chariots utilisés en secteur hospitalier, mais également, par exemple les poignées de porte ou tous les objets susceptibles d'être contaminés par contact et de devenir ainsi contaminants, ces propriétés biocides et/ou bactériostatiques de contact sont susceptibles de présenter un grand intérêt.

Pour les industriels du secteur des colles ou des peintures, ou les fabricants de produits cosmétiques par exemple, on recherchera des propriétés biocide et/ou bactériostatique pour l'amidon et les matrices cellulosiques.

Par bactériostatique, on entend la propriété qui consiste à inhiber le développement des micro-organismes et notamment les bactéries.

Plus précisément les substrats présentant cette propriété bactériostatique de contact, sont susceptibles d'inhiber le développement des micro-organismes par simple contact. Ceux présentant intrinsèquement cette propriété, comme de l'amidon modifié, et des matrices cellulosiques modifiées permettent d'inhiber le développement des microorganismes entrant à leur contact, par exemple par contamination accidentelle, sans avoir recours à l'addition de « conservateurs ».

Par biocide, on entend la propriété qui consiste à détruire un microorganisme tel que champignon, levure ou bactérie.

Plus précisément, les substrats présentant cette propriété biocide de contact sont susceptibles d'entraîner la mort d'un microorganisme par simple contact. Ceux présentant intrinsèquement cette propriété, comme de l'amidon modifié, et des matrices cellulosiques modifiées, permettent d'entraîner la mort de tous microorganismes susceptibles de les contaminer, sans avoir recours à des additifs spécifiques comme les conservateurs et les désinfectants.

Les procédés utilisés pour modifier les substrats et leur conférer les propriétés particulières recherchées, ne doivent pas dégrader les propriétés propres à ces substrats, par exemple, lorsque ces substrats sont des fibres textiles leurs propriétés absorbantes, lorsque ces substrats sont des préparations cellulosiques destinées à être mises en oeuvre sous forme de films d'emballage leur résistance mécanique, et lorsqu'il s'agit d'amidon destiné à une préparation de colle ou de peinture ses propriétés épaississantes.

Pour les objets, si ceux-ci ne sont pas protégés par enduction d'une couche de peinture ou par un revêtement présentant les caractéristiques recherchées, le greffage ne devra pas modifier les propriétés physiques de la masse, par exemple dureté, résistance mécanique, et/ou les propriétés de surface tant physiques qu'esthétiques.

La protection de substrats, notamment textiles ou emballages, contre des agents tels que des bactéries, champignons ou levures, par traitement desdits substrats avec des composés ou produits présentant une activité spécifique vis à vis de ces microorganismes, est déjà bien décrite.

Ces techniques déjà décrites et utilisées font appel à des procédés de traitement qui peuvent être scindés en deux catégories :
- d'une part, les procédés mettant en oeuvre des moyens d'ennoblissement par trempage, enduction ou tout autre moyen qui sera assimilable à une imprégnation, imbibition ou dépôt suivi d'un simple séchage, c'est à dire sans modification de la structure chimique des constituants, ni création de liaisons chimiques covalentes,
- et d'autre part, les procédés mettant en oeuvre des moyens d'ennoblissement par greffage de composés actifs, par modification de la structure chimique des substrats et création de liaisons chimiques entre le substrat et le composé présentant les propriétés recherchées.

Les techniques dites d'imprégnation sont mises en oeuvre par adjonction de produits présentant les propriétés recherchées, par exemple dans le dernier bain de rinçage.

Ces techniques peuvent présenter trois inconvénients majeurs dus au caractère temporaire de l'imprégnation effectuée et du relargage possible des produits d'ennoblissement :
- la nécessité de renouveler l'ennoblissement après un usage prolongé ou le lavage,
- un risque de mauvaise pénétration des produits d'ennoblissement,
- des risques potentiels d'intolérance et d'allergie chez certains sujets.

Les techniques par greffage covalent permettent d'obtenir des produits ne présentant pas les inconvénients précités, mais, les procédés mis en oeuvre ou les produits utilisés ne sont pas économiquement viables et concurrentiels vis à vis des techniques par imprégnation.

On connaît par le document FR-A-2511001 la mise en oeuvre d'un procédé par greffage, par traitement de polymères polyhydroxylés ou polyaminés par des sels de n-oxiraneméthane N,N,N -trialkylaminium de formule : 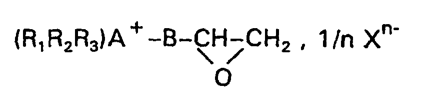 dans laquelle :
- A présente un atome d'azote ou de phosphore
- R₁, R₂, sont choisis dans le groupe constitué par des radicaux alkyle de C₁ à C₂₀, ou dans le groupe constitué par des radicaux alkényle de C₁ à C₂₀ et des radicaux aryle éventuellement substitués,
- R₁ et R₂ pouvant éventuellement former un hétérocycle avec A, lorsque A est de l'azote
- R₃ est choisi dans le groupe constitué par les radicaux alkyles de C1 à C20, les radicaux alkényle de C1 à C20, les radicaux aryle éventuellement substitués et le radical 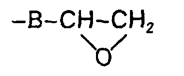
- B est un radical alkylène compris entre C₁ et C₁₀
- Xⁿ⁻ est un anion
- n est un nombre valant 1, 2 ou 3

Dans ce document, le procédé de synthèse des sels de N-oxiraneméthane N,N,N-trialkylaminium a un rendement relativement faible de l'ordre de 60 à 70 %.

Les composés décrits dans le document français FR-A-251 1001 sont par exemple des N-oxiraneméthanes, et aucun textile traité avec ces composés n'existe aujourd'hui sur le marché, à la meilleure connaissance du Demandeur.

La solution développée était pourtant déjà une réponse aux problèmes résultant de l'utilisation des techniques d'imprégnation.

Selon le document FR-A-2695800, on a décrit un procédé de greffage covalent, par activation radicalaire sous l'effet d'un rayonnement ionisant tel qu'un rayonnement gamma.

Cette solution par activation radicalaire par rayonnement gamma d'un groupe fonctionnel éthylénique, en remplacement du groupe époxyde, est motivée notamment par la difficulté de contrôler une réaction mettant en oeuvre des composés comportant des groupes époxydes, tels que décrits dans le document FR-A-FR2695800 cité précédemment, et par le faible rendement du procédé de synthèse des réactifs mis en oeuvre.

Mais, la fabrication à l'échelle industrielle d'un textile biocide par le procédé tel que décrit, et plus particulièrement l'étape de greffage du composé biocide sur le substrat avec un rayonnement ionisant tel que le rayonnement gamma, représente un investissement technologique très lourd, et n'est pas économiquement viable.

Selon le document FR-A-2781489, les inconvénients précités de l'art antérieur ont été surmontés par la mise en oeuvre d'un procédé d'enduction, sans greffage covalent, avec un copolymère d'acrylamide ou de dérivés d'acrylamide et des sels de trialkylammonium connus pour leur activité biocide.

Les impératifs économiques sont satisfaits par la mise en oeuvre de ces composés, mais le caractère biocide ou bactéricide des substrats ainsi traités ne présente pas le caractère permanent recherché.

On sait que certains sels de trialkylammmonium constituent une classe de composés possédant des propriétés nettoyantes et désinfectantes.

La Demanderesse a découvert une famille de nouveaux sels de trialkylammonium, biocides ou bactériostatiques, comportant une fonction époxyde, permettant un greffage par liaison covalente, sur le substrat par ouverture de la fonction époxyde en milieu basique.

Ces nouveaux sels de trialkylammonium comportant une fonction époxyde sont facilement accessibles avec de très bons rendements, par l'action des oxydants de type peracides ou peroxydes, et conservent leurs propriétés biocides après greffage.

Un composé de l'invention consiste en un sel de trialkylammonium de formule générale **( I )** : 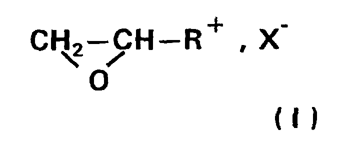 dans laquelle :
R représente un groupe :

   **-(CH**_{**2**}**)**_{**n**}**-A-NH-(CH**_{**2**}**)**_{**n'**}**-N**^{**+**}**R**_{**1**}**R**_{**2**}**R**_{**3**}

   dans lequel :
   A représente un groupe -CO- ou -CH₂-
   n' et n sont compris chacun entre 1 et 20, avec (n+n') supérieur ou égal à 10,
   R₁,R₂ et R₃ sont des groupes alkyle ou mono-hydroxyalkyle en C1 à C20, linéaires ou ramifiés, identiques ou différents.
X⁻ représente le contre-ion.

Un composé préféré selon l'invention est le composé de formule **(II)**

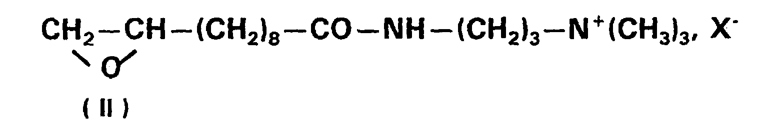

Un autre objet de l'invention est le procédé de synthèse de ces sels de trialkylammonium par réaction d'époxydation en une seule étape sur des composés de formule générale **( III )** :

**CH**_{**2**} **= CH-R**^{**+**}**,X**^{**-**} **(III)**

- dans laquelle R représente un radical de formule générale telle que définie ci-dessus dans **( I )**,
- par action d'oxydants de type peracides ou peroxydes en solution aqueuse ou organique.

On citera à titre d'exemple :
- les peracides de formule générale R'COOOH décrits notamment dans Org. Réactions 1953,7,378, et de préférence l'acide métachloroperoxybenzoïque, seul ou en association avec une base comme NaHCO₃, Na₂CO₃, K₂CO₃, ou un phosphate,
- les peroxydes comme le peroxyde d'hydrogène H₂O₂ en présence d'un catalyseur métallique, comme les catalyseurs à base de fer, de molybdène ou de manganèse et de préférence le tungstène sous forme d'acide, de sels ou d'oxydes.

Un autre objet de l'invention est le procédé de greffage des sels de trialkylammonium comportant une fonction époxyde de formule I par réaction avec les fonctions chimiques comportant des hydrogènes labiles des macromolécules constitutives des substrats destinés à être soumis au greffage.

Les fonctions comportant des hydrogènes labiles sont les fonctions -OH, -SH, >NH, -CO(NH)-, ou -COOH, et le taux de greffage sera dépendant du taux de fonctionnalisation du substrat.

Parmi les substrats, comportant des ramifications fonctionnalisées acide, alcool, thiol, amide ou amine, on citera, à titre d'exemple et de façon non limitative, les matériaux polyosiques, les matériaux polyamides, les polyuréthannes ou les polyimines, les polyurées, les résines formophénoliques, et tous les matériaux produits par homopolymérisation ou copolymérisation de monomères du type acide acrylique ou méthacrylique ou leurs dérivés esters, acide vinylacétique et ses dérivés esters, acide butènedioïque et ses dérivés esters, acide itaconique et ses dérivés esters, acide vinylsulfonique et ses dérivés esters, alcool allylique et ses dérivés esters, alcool vinylique estérifié et les polyvinyl alcools et leurs dérivés acrylamide ou métacrylamide, mercaptoéthylène.

Par matériaux polyosiques, on entend les matériaux constitués d'enchaînements de plusieurs oses.

La plupart d'entre eux sont naturels, et le principal est la cellulose et ses dérivés, mais aussi l'amidon. On citera à titre d'illustration les fibres naturelles et végétales comme le coton, le kapok, le lin, le chanvre, la ramie et le jute, les fibres régénérées, modifiées ou synthétiques comme la viscose, la rayonne, le meryl, le zantrel et la cellophane, utilisées pour les textiles et l'emballage, mais aussi le bois, le carton, le papier utilisé en ameublement et en emballage.

Les substrats modifiés selon l'invention ont des applications également en cosmétologie, notamment les matrices cellulosiques, et l'amidon dans l'industrie des colles et des peintures.

Par matériaux polyamides, on entend des matériaux, qui incluent des fonctions amides dans la chaîne polymérique, dont certains sont naturels, par exemple les polyaminoacides ou protéines.

Parmi les polyamides naturels, on citera à titre d'illustration la laine, la soie, la kératine.

Parmi les polyamides synthétiques, on citera principalement la famille des polyamides 6-6, par exemple le Nylon ®.

Ce procédé selon l'invention comprend les étapes de traitement du substrat à greffer, en milieu basique en solution aqueuse, puis par une solution de sel de trialkylammonium selon la formule (I).

Par exemple, pour le greffage sur cellulose le schéma réactionnel est le suivant :

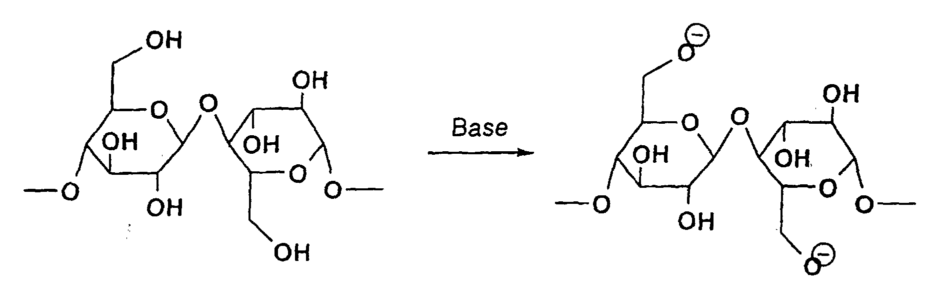

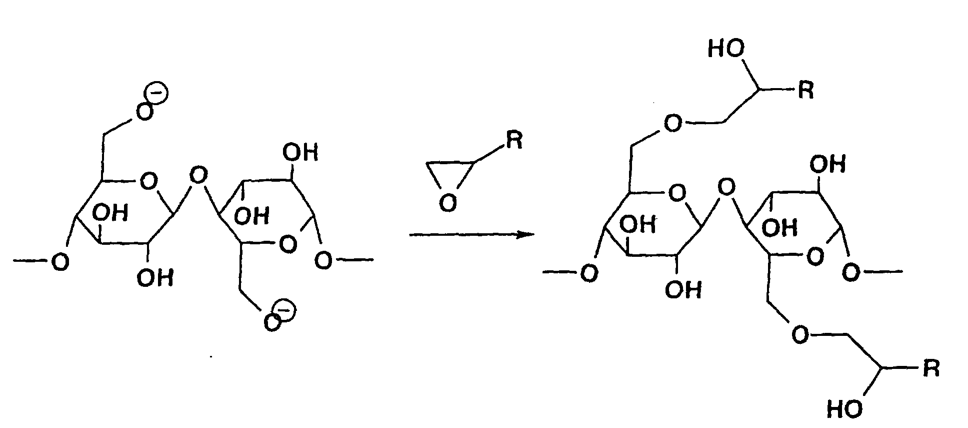

R représente un radical tel que défini dans la formule générale I.

Les caractéristiques et objets de l'invention sont mis en évidence dans les exemples suivants :

### Exemple 1 : Préparation d'une solution aqueuse basique d'époxyde à 25% massique

A 40 g d'une solution aqueuse à 50% massique de méthylsulfate de triméthylammonium undécyl-amide-propyle (CAS Registry Number : 94313-91-4) (0,051 moles) sont ajoutés 13,66 g de NaHCO₃ (0,163 moles). On additionne ensuite goutte à goutte (1,5 heures) une solution de 14,02 g (0,081 moles) d'acide méta-chloroperbenzoïque dissous dans 530 ml de dichlorométhane (concentration : 0,15 M). La solution est maintenue 48 heures sous vive agitation. Après décantation et lavage du matériel par 40 ml d'eau, la phase aqueuse est lavée une fois par 100 ml d'éther diéthylique pour éliminer les traces d'acide méta-chlorobenzoïque, dissoutes dans la phase aqueuse.

La solution obtenue a une concentration massique de 25%. Elle présente en RMN du proton (D₂O) 18% de l'alcène de départ (multiplets à 5,8 et 4,95 ppm) et 82% d'époxyde caractérisé par les multiplets à 3,7 ppm, 2,67 ppm et 2,92 ppm.

Les tests biologiques de cette solution aqueuse basique d'époxyde à 25% massique ont donné une activité similaire à celle du produit de départ à 50% dans l'eau.

### Exemple 2 : Préparation d'une solution aqueuse neutre d'époxyde à 25% massique

A 20 g d'une solution aqueuse à 50% massique de méthylsulfate de triméthylammonium undécyl-amide-propyle (CAS Registry Number :94313-91-4) (0,025 moles) est additionnée goutte à goutte (2,5 heures) une solution de 7,01 g (0,041 moles) d'acide méta-chloroperbenzoïque dissous dans 260 ml de dichlorométhane (concentration : 0,16 M). La solution est maintenue 48 heures sous vive agitation. Après décantation et lavage du matériel par 20 ml d'eau, la phase aqueuse est lavée une fois par 100 ml d'éther diéthylique pour éliminer les traces d'acide méta-chlorobenzoïque, dissoutes dans la phase aqueuse.

La solution obtenue a une concentration massique de 25%. Elle ne présente plus en RMN du proton (D₂O) de trace de l'alcène de départ (multiplets à 5,8 et 4,95 ppm). L'époxydation a donc un rendement quasi quantitatif.

L'époxyde est caractérisé en RMN du proton dans D₂O par les multiplets à 3,7 ppm, 2,67 ppm et 2,92 ppm.

### Exemple 3 : Préparation d'une solution aqueuse neutre d'époxyde à 50% massique

A 112g d'une solution aqueuse à 50% massique de méthylsulfate de triméthylammonium undécyl-amide-propyle (CAS Registry Number :94313-91-4) (0,198 moles) est additionnée goutte à goutte (1,5 heures) une solution de 53,93 g (0,313 moles) d'acide méta-chloroperbenzoïque dissous dans 1l de dichlorométhane (concentration : 0,3 M). La solution est maintenue 48 heures sous vive agitation. Après décantation, la phase aqueuse est lavée une fois par 200 ml d'éther diéthylique pour éliminer les traces d'acide méta-chlorobenzoïque, dissoutes dans la phase aqueuse.

La solution obtenue ne présente plus en RMN du proton (D₂O) de trace de l'alcène de départ (multiplets à 5,8 et 4,95 ppm). L'époxydation a donc un rendement quasi quantitatif.

L'époxyde est caractérisé en RMN du proton dans D₂O par les multiplets à 3,7 ppm, 2,67 ppm et 2,92 ppm.

Les tests biologiques de cette solution aqueuse d'époxyde à 50% massique ont donné une activité similaire à celle du produit de départ à 50% dans l'eau.

### Exemple 4 : Greffage d'un fil de coton à l'aide d'une solution aqueuse basique d'époxyde à 25% massique

Le fil de coton est préalablement traité dans de la soude aqueuse à 1,5% massique à ébullition pendant 3 heures. Le fil est alors lavé à l'eau jusqu'à neutralité, puis séché à l'étuve 48 heures à 65 °C.

1,986 g de coton traité sec sont placés dans une bombe à agitation mécanique verticale dans 31,8 ml d'isopropanol en présence de 3,22 ml de soude aqueuse à 15,8 % massique. L'agitation est maintenue à température ambiante pendant 1 heure.

A la solution sont alors ajoutés 16,05 g de la solution aqueuse basique d'époxyde à 25% (exemple 1) et 2 ml d'isopropanol. La solution est agitée et chauffée à 65°C pendant 5 heures.

Après refroidissement à température ambiante, le fil est essoré puis séché à l'étuve à 65°C pendant 48 heures.

### Exemple 5: Greffage d'un fil de coton à l'aide d'une solution aqueuse neutre d'époxyde à 25% massique

Le fil de coton est préalablement traité dans de la soude aqueuse à 1,5% massique à ébullition pendant 3 heures. Le fil est alors lavé à l'eau jusqu'à neutralité, puis séché à l'étuve 48 heures à 65 °C.

1,44 g de coton traité sec sont placés dans une bombe à agitation mécanique verticale dans 23,1 ml d'isopropanol en présence de 2,34 ml de soude aqueuse à 15,8 % massique. L'agitation est maintenue à température ambiante pendant 1 heure.

A la solution sont alors ajoutés 11,64 g de la solution aqueuse d'époxyde à 25% (exemple 2) et 1,45 ml d'isopropanol. La solution est agitée et chauffée à 65°C pendant 5 heures.
Après refroidissement à température ambiante, le fil est essoré puis séché à l'étuve à 65°C pendant 48 heures.

### Exemple 6: Greffage d'un fil de coton à l'aide d'une solution aqueuse neutre d'époxyde à 50% massique

Le fil de coton est préalablement traité dans de la soude aqueuse à 1,5% massique à ébullition pendant 3 heures. Le fil est alors lavé à l'eau jusqu'à neutralité, puis séché à l'étuve 48 heures à 65°C.

1,477 g de coton traité sec sont placés dans une bombe à agitation mécanique verticale dans 24 ml d'isopropanol en présence de 2,4 ml de soude aqueuse à 15,8 % massique. L'agitation est maintenue à température ambiante pendant 1 heure.

A la solution sont alors ajoutés 5,97 g de la solution aqueuse basique d'époxyde à 50% (exemple 3) et 1,5 ml d'isopropanol. La solution est agitée et chauffée à 65°C pendant 5 heures.

Après refroidissement à température ambiante, le fil est essoré puis séché à l'étuve à 65°C pendant 48 heures.

### Exemple 7 : Greffage d'un fil de coton à l'aide d'une solution neutre d'époxyde à 142,5 g/l dans l'isopropanol

Le fil de coton est préalablement traité dans de la soude aqueuse à 1,5% massique à ébullition pendant 3 heures. Le fil est alors lavé à l'eau jusqu'à neutralité, puis séché à l'étuve 48 heures à 65 °C.

1,038 g de coton traité sec sont placés dans une bombe à agitation mécanique verticale dans 17 ml d'isopropanol en présence de 1,7 ml de soude aqueuse à 15,8 % massique. L'agitation est maintenue à température ambiante pendant 1 heure.

A la solution sont alors ajoutés 14,8 g d'une solution neutre d'époxyde évaporé (exemple 3) dilué à 142,5 g/l dans de l'isopropanol. La solution est agitée et chauffée à 65 °C pendant 5 heures.

Après refroidissement à température ambiante, le fil est essoré puis séché à l'étuve à 65°C pendant 48 heures.

### Exemple 8 : Greffage d'un fil de coton à l'aide d'une solution aqueuse neutre d'époxyde à 50 % massique en présence de 1,2 propanediol (MPG).

Le fil de coton est préalablement traité dans de la soude aqueuse à 1,5 % massique à ébullition pendant 3 heures. Le fil est alors lavé à l'eau jusqu'à neutralité, puis séché à l'étuve 48 heures à 65 °C.

1,753 g de coton traité sec sont placés dans une bombe à agitation mécanique verticale dans 28 ml de 1,2-propane diol (MPG) en présence de 2,8 ml de soude aqueuse à 15,8 % massique. L'agitation est maintenue à température ambiante pendant 1 heure.

A la solution sont alors ajoutés 7,08 g d'une solution aqueuse d'époxyde à 50 % (exemple 3) et 1,8 ml de MPG. La solution est agitée et chauffée à 65°C pendant 5 heures.

Après refroidissement à température ambiante, le fil est essoré puis séché à l'étuve à 65°C pendant 48 heures.

### Exemple 9 : Greffage d'un tricot de coton blanc à l'aide d'une solution aqueuse neutre d'époxyde à 50 % massique

1,104 g de tricot coton blanc sont placés dans une bombe à agitation mécanique verticale dans 18 ml d'isopropanol en présence de 1,8 ml de soude aqueuse à 15,8 % massique. L'agitation est maintenue à température ambiante pendant 1 heure.

A la solution sont alors ajoutés 4,47 g d'une solution aqueuse d'époxyde à 50 % (exemple 3) et 1,2 ml d'isopropanol. La solution est agitée et chauffée à 65°C pendant 5 heures.

Après refroidissement à température ambiante, le tricot est essoré puis séché à l'étuve à 65°C pendant 48 heures. Le tricot obtenu montre un effet bactéricide de contact selon les deux tests bactériologiques spécifiques aux tricots, décrits à l'exemple 15.

### Exemple 10 : Greffage d'un tricot de coton brut à l'aide d'une solution aqueuse neutre d'époxyde à 50 % massique

Le tricot de coton brut est préalablement traité dans de la soude aqueuse à 1,5 % massique à ébullition pendant 3 heures. Le tricot est alors lavé à l'eau jusqu'à neutralité, puis séché à l'étuve 48 heures à 65°C.

1,157 g de tricot de coton traité sec sont placés dans une bombe à agitation mécanique verticale dans 19 ml d'isopropanol en présence de 1,9 ml de soude aqueuse à 15,8 % massique. L'agitation est maintenue à température ambiante pendant 1 heure.

A la solution sont alors ajoutés 4,67 g d'une solution aqueuse d'époxyde à 50 % (exemple 3) et 1,2 ml d'isopropanol. La solution est agitée et chauffée à 65°C pendant 5 heures.

Après refroidissement à température ambiante, le tricot est essoré puis séché à l'étuve à 65°C pendant 48 heures. Le tricot obtenu montre un effet bactéricide de contact selon les deux tests bactériologiques spécifiques aux tricots, décrits à l'exemple 15.

### Exemple 11 : Greffage d'un fil de viscose à l'aide d'une solution aqueuse neutre à 50 % massique

1 ,420 g de fil de viscose traité sec sont placés dans une bombe à agitation mécanique verticale dans 23 ml d'isopropanol en présence de 2,3 ml de soude aqueuse à 15,8 % massique.

L'agitation est maintenue à température ambiante pendant 1 heure.

A la solution sont alors ajoutés 5,74 g d'une solution aqueuse d'époxyde à 50 % (exemple 3) et 1,5 ml d'isopropanol. La solution est agitée et chauffée à 65°C pendant 5 heures.

Après refroidissement à température ambiante, le fil est essoré puis séché à l'étuve à 65 °C pendant 48 heures.

### Exemple 12 : Greffage d'un fil de laine naturelle à l'aide d'une solution aqueuse neutre d'époxyde à 50 % massique sans traitement basique préalable.

1,618 g de fil de laine naturelle sont placés dans une bombe à agitation mécanique verticale dans 26 ml. L'agitation est maintenue à température ambiante pendant 1 heure.

A la solution sont alors ajoutés 6,472 g d'une solution aqueuse d'époxyde à 50 % (exemple 3) et 1,6 ml d'isopropanol. La solution est agitée et chauffée à 40°C pendant 5 heures.

Après refroidissement à température ambiante, le fil est essoré puis séché à l'étuve à 65 °C pendant 48 heures.

### Exemple 13 : Greffage d'un fil de laine naturelle à l'aide d'une solution aqueuse neutre d'époxyde à 50 % massique avec traitement basique préalable

1,856 g de fil de laine naturelle sont placés dans une bombe à agitation mécanique verticale dans 30 ml avec 3 ml d'une solution aqueuse de carbonate de potassium (K₂CO₃) à 10 % massique. L'agitation est maintenue à température ambiante pendant 1 heure.

A la solution sont alors ajoutés 7,424 g d'une solution aqueuse d'époxyde à 50 % (exemple 3) et 1,9 ml d'isopropanol. La solution est agitée et chauffée à 40°C pendant 5 heures.

Après refroidissement à température ambiante, le fil est essoré puis séché à l'étuve à 65°C pendant 48 heures.

### Exemple 14 : Résultats et tests d'activité biologique

Le principe de caractérisation est le dépôt d'une suspension bactérienne sur un morceau d'échantillon à analyser et le dénombrement des bactéries viables après un temps préalablement établi de contact, en comparaison avec un témoin non traité.

La souche utilisée est *Staphylococcus aureus*.

L'échantillon à tester est placé dans une fiole stérile puis est déposé sur cet échantillon 0,4 ml de suspension de bactérie à 4,5.10⁵ bactéries/cm³.

Le contact est maintenu à 30 ±1 °C pendant 24 heures entre l'échantillon et la solution bactérienne.

Après l'expiration du temps de contact, on verse 10 ml de diluant (tryptine : 1g ; NaCl : 8,5 g ; eau : QSP 1 l) dans la fiole sous forte agitation pour décrocher les bactéries du substrat.

Le dénombrement des bactéries viables est alors effectué par la méthode du nombre le plus probable (NPP) de bactéries.

Cette manipulation doit se faire sur quatre échantillons identiques en présence d'un témoin ne renfermant aucune molécule biocide.

Soit Nₜ la moyenne des colonies dénombrées, soit N₀ la moyenne des colonies déposées sur l'échantillon au temps T₀ (4,5.10⁵ bactéries/cm³), l'activité est définie par le logarithme du rapport Nₜ/N₀ (réduction logarithmique).

Les résultats obtenus sur quatre échantillons avant lavage et après lavages sont rassemblés dans le tableau 1.

On notera la permanence de l'activité notamment pour l'échantillon n°4, pour lequel après 20 lavages, l'activité ne subit aucune altération.

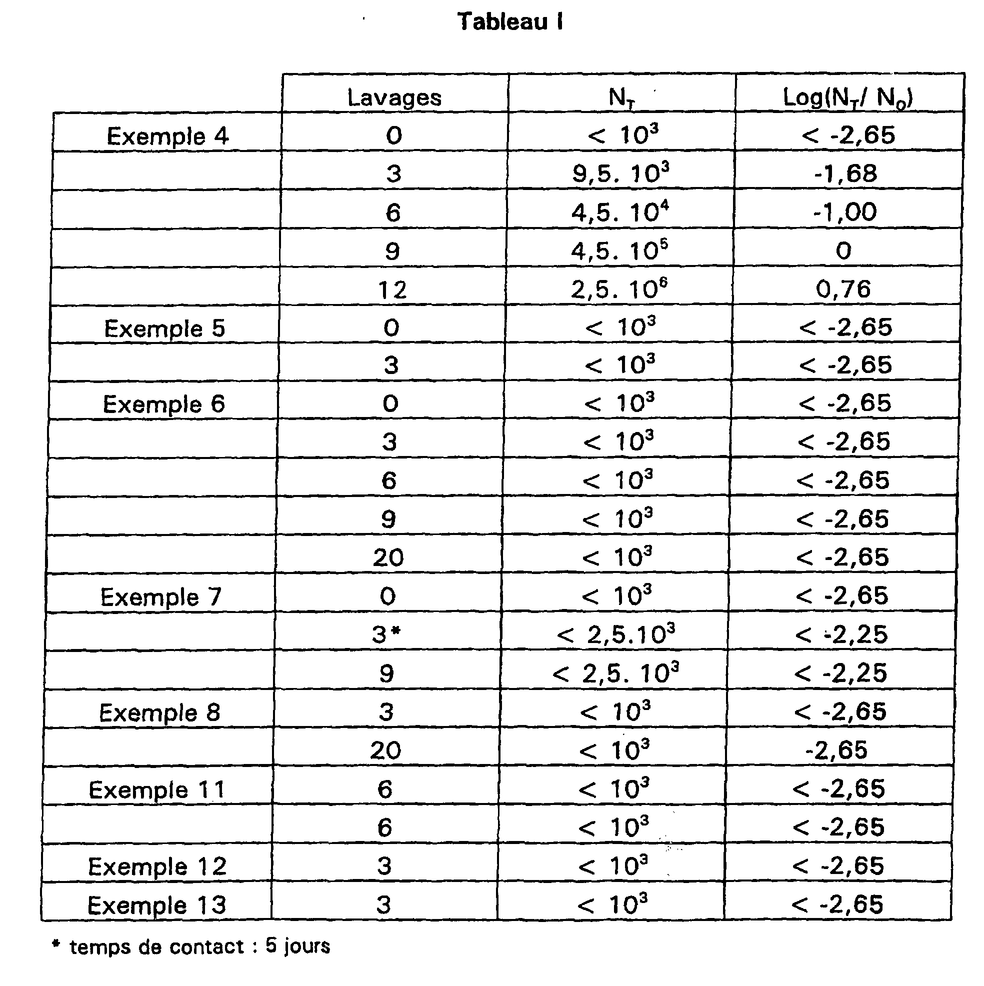

### Exemple 15 : Tests d'activité biologique pour les tricots

Les tests sont effectués avec la bactérie *Staphylococcus aureus* en boîte de pétri. Deux sortes de tests bactériologiques ont été utilisés. On découpe dans la surface du textile à tester des disques de 3 cm de diamètre. Le test est à effectuer sur trois éprouvettes. Afin d'éviter des contaminations sur la gélose (bactéries, champignons divers), les échantillons sont stérilisés.

### Test 1 :

L'éprouvette à tester (témoin ou traitée) est placée sur de la gélose nutritive inoculée avec les bactéries de test prévues (0,1 ml à 10⁵ bact/ml) dans une boîte de pétri.

L'éprouvette traitée : l'effet antibactérien du traitement se manifeste après l'incubation à 30°C par le fait que la zone de la plaque de gélose nutritive qui était en contact avec l'éprouvette (zone de contact) est exempte de bactéries. On peut parfois constater une zone additionnelle sans développement de bactéries autour du bord de l'éprouvette. C'est la zone d'inhibition qui dénote un certain départ de l'antiseptique. Elle démontre la « non-solidité » de la fixation du biocide dans la fibre.

L'éprouvette témoin : on observe une invasion bactérienne importante sous l'éprouvette témoin, sans zone d'inhibition.

### Test 2 :

0,1 ml de bouillon de culture contenant environ 10⁵ bact/ml est déposé sur l'éprouvette à tester (témoin ou traitée), elle-même sur une boîte de pétri vide stérile. Il faut veiller à une bonne pénétration de la goutte dans l'éprouvette : il s'avère parfois nécessaire d'ajouter un mouillant au bouillon de culture.

On dépose aussitôt l'éprouvette sur de la gélose stérile (t=0h), la boîte de pétri est ensuite placée à l'étuve à 30°C pendant 24 heures.

On peut également attendre 24 heures avant de déposer l'éprouvette sur la gélose stérile (t=24h). On laisse incuber pendant 24 heures à l'étuve à 30°C.

L'effet antibactérien se manifeste, après cette période d'incubation, par l'absence de développement bactérien sous l'éprouvette traitée. On constate un développement bactérien important sous le témoin.

### Exemple 16 : Protocole de lavage des échantillons pour mimer l'action du lavage

A environ 1,5 g de fibre (ou de tricot) placés dans un appareil à agitation verticale sont additionnés 100 ml d'eau déminéralisée.

L'agitation est maintenue pendant 5 minutes puis la fibre (ou le tricot) est essorée.

### Exemple 17 : Greffage d'un tissu de coton en machine industrielle, à l'aide d'une solution aqueuse neutre d'époxyde à 50% massique.

En vue d'un développement ultérieur, le greffage sur un tissu de coton blanc a été effectué sur une machine pilote de type Jigger. Les résultats obtenus sur ce type de machine sont directement transportables au niveau industriel.

Le tissu greffé est une bande de coton blanc de 0,25 m de largeur sur 5 m de longueur soit une surface totale de 1,25 m². Le tissu pèse 162,5 g par m², le traitement est donc effectué avec un volume de bain de 3L selon le protocole ci-dessous.

Le bain est rempli par 0,3 L de soude aqueuse à 16% massique et 2,7 L de 1,2 propanediol. Le tissu est mis en circulation à vitesse moyenne pendant 1 heure.

Le bain est vidangé puis rempli avec 650 g d'époxyde à 50% dans l'eau et 2,3 L de 1,2 propanediol. Le tissu est mis en circulation à vitesse moyenne en même temps que le chauffage (T = 0). La température de consigne (65°C) est atteinte en 10 minutes puis maintenue pendant 4 heures.

Les prélèvements effectués à T = 10 mn, 1 h10', 2h40' et 4h10, incluent la portion de tissu n'ayant pas baignée (comprise entre le bain et le rouleau).

Les échantillons prélevés à 1 h10', 2h40' et 4h10 montrent une activité biocide de contact. Le traitement est donc efficace dès 1h.

L'absence de zone d'inhibition autour de l'échantillon permet de visualiser l'absence de relargage du principe actif.

## Revendications

1. Sel de trialkylammonium époxydé, **caractérisé en ce qu'**il est choisi parmi les sels de formule générale **( I )** : 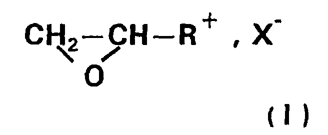 dans laquelle :
R représente un groupe :
**-(CH**_{**2**}**)**_{**n**}**-A-NH-(CH**_{**2**}**)**_{**n'**}**-N** ^{**+**}**R**_{**1**}**R**_{**2**}**R**_{**3**}
dans lequel :
A représente un groupe -CO- ou -CH₂-
n' et n sont compris chacun entre 1 et 20, avec (n+n') supérieur ou égal à 10,
R₁,R₂ et R₃ sont des groupes alkyles ou mono-hydroxyalkyles en C₁ à C₂₀, linéaires ou ramifiés, identiques ou différents.
X⁻ représente le contre-ion.

2. Sel de trialkylammmonium époxydé, selon la revendication 1, **caractérisé en ce que** -R représente le radical :
**-(CH**_{**2**}**)**_{**8**}**-CO-NH-(CH**_{**2**}**)**_{**3**}**-N**^{**+**}**(CH**_{**3**}**)**_{**3**}

3. Procédé de synthèse de sels de trialkylammonium époxydés selon la revendications 1 ou 2, **caractérisé en ce qu'**il consiste à faire réagir un composé de formule générale CH2 = CH-R⁺,X⁻ dans laquelle R représente un groupe :
**-(CH**_{**2**}**)**_{**n**}**-A-NH-(CH**_{**2**}**)**_{**n'**}**-N**^{**+**}**R**_{**1**}**R**_{**2**}**R**_{**3**}
dans lequel :
A représente un groupe -CO- ou -CH₂-
n' et n sont compris entre 1 et 20, avec (n+n') supérieur ou égal à 10,
R₁,R₂ et R₃ sont des groupes alkyle ou mono-hydroxyalkyle en C1 à C4 linéaires ou ramifiés, identiques ou différents.
X⁻ représente le contre-ion.
Avec un oxydant de type peracides ou peroxydes en solution aqueuse ou organique.

4. Procédé de greffage covalent des sels de trialkylammonium selon la revendication 1 ou 2, **caractérisé en ce qu'**il comprend les étapes de traitement d'un substrat à greffer en milieu basique en solution aqueuse ou organique, puis traitement par une solution de sels de trialkylammonium selon la revendication 1 ou 2.

5. Procédé selon la revendication 4, **caractérisé en ce que** les macromolécules du substrat comportent des hydrogènes labiles.

6. Substrat biocide susceptible d'être obtenu par un procédé de greffage selon la revendication 4.

7. Utilisation des sels de trialkylammonium selon les revendications 1 et 2 pour obtenir un substrat biocide ou bactériostatique par contact.

## Claims

1. Epoxidized trialkylammonium salt, **characterized in that** it is chosen from the salts of general formula (I) : 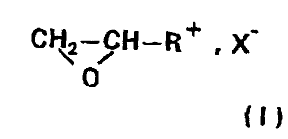 in which:
R represents a group:
**-(CH**_{**2**}**)**_{**n**}**-A-NH-(CH**_{**2**}**)**_{**n'**}**-N**^{**+**}**R**_{**1**}**R**_{**2**}**R**_{**3**}
in which:
A represents a group -CO- or -CH₂-
n' and n are each between 1 and 20, with (n+n') greater than or equal to 10,
R₁, R₂ and R₃ are linear or branched C₁ to C₂₀ alkyl or monohydroxyalkyl groups, which may be identical or different,
X⁻ represents the counter-ion.

2. Epoxidized trialkylammonium salt according to Claim 1, **characterized in that** -R represents the radical:
**-(CH**_{**2**}**)**_{**8**}**-CO-NH-(CH**_{**2**}**)**_{**3**}**-N**^{**+**}**(CH**_{**3**}**)**_{**3**}

3. Method for synthesizing epoxidized trialkylammonium salts according to Claim 1 or 2, **characterized in that** it consists in reacting a compound of general formula CH2=CH-R⁺,X⁻ in which R represents a group:
**-(CH**_{**2**}**)**_{**n**}**-A-NH-(CH**_{**2**}**)**_{**n'**}**-H**^{**+**}**R**_{**1**}**R**_{**2**}**R**_{**3**}
in which:
A represents a group -CO- or -CH₂-
n' and n are between 1 and 20, with (n+n') greater than or equal to 10,
R₁, R₂ and R₃ are linear or branched C1 to C4 alkyl or monohydroxyalkyl groups, which may be identical or different,
X⁻ represents the counter-ion,
with an oxidant of the peracid or peroxide type in aqueous or organic solution.

4. Method for covalent grafting of the trialkylammonium salts according to Claim 1 or 2, **characterized in that** it comprises the steps of treating a substrate to be grafted in a basic medium in an aqueous or organic solution, and then treating with a solution of trialkylammonium salts according to Claim 1 or 2.

5. Method according to Claim 4, **characterized in that** the macromolecules of the substrate contain labile hydrogens.

6. Biocidal substrate which can be obtained by a method of grafting according to Claim 4.

7. Use of the trialkylammonium salts according to Claims 1 and 2 for obtaining a substrate which is biocidal or bacteriostatic by contact.

## Patentansprüche

1. Epoxidgruppenhaltiges Trialkylammoniumsalz, **dadurch gekennzeichnet, daß** es unter Salzen der allgemeinen Formel (I): 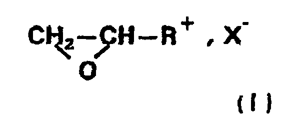 ausgewählt ist, worin:
R für eine Gruppe:
**-(CN**_{**2**}**)**_{**n**}**-A-NH-(CH**_{**2**}**)**_{**n'**}**-N**^{**+**}**R**_{**1**}**R**_{**2**}**R**_{**3**}
steht, worin:
A für eine -CO- oder -CH₂-Gruppe steht,
n und n' jeweils einen Wert zwischen 1 und 20 haben, wobei (n+n') größer gleich 10 ist,
R₁, R₂ und R₃ für gleiche oder verschiedene, lineare oder verzweigte C₁-C₂₀-Alkyl- oder C₁-C₂₀-Monohydroxyalkylgruppen stehen,
X⁻ für das Gegenion steht.

2. Epoxidgruppenhaltiges Trialkylammoniumsalz, nach Anspruch 1, **dadurch gekennzeichnet, daß** -R für den Rest:
**-(CH**_{**2**}**)**_{**8**}**-CO-NH-(CH**_{**2**}**)**_{**3**}**-N**^{**+**}**(CH**_{**3**}**)**_{**3**}
steht.

3. Verfahren zur Synthese von epoxidgruppenhaltigen Trialkylammoniumsalzen nach Anspruch 1 oder 2, **dadurch gekennzeichnet, daß** man eine Verbindung der allgemeinen Formel CH₂=CH-R⁺,X⁻, worin R für eine Gruppe:
**-(CH**_{**2**}**)**_{**n**}**-A-NH-(CH**_{**2**}**)**_{**n'**}**-N**^{**+**}**R**_{**1**}**R**_{**2**}**R**_{**3**}
steht, worin:
A für eine -CO- oder -CH₂-Gruppe steht,
n und n' jeweils einen Wert zwischen 1 und 20 haben, wobei (n+n') größer gleich 10 ist,
R₁, R₂ und R₃ für gleiche oder verschiedene, lineare oder verzweigte C₁-C₄-Alkyl- oder C₁-C₄-Monohydroxyalkylgruppen stehen,
X⁻ für das Gegenion steht,
in wäßriger oder organischer Lösung mit einem Oxidationsmittel vom Persäure- oder Peroxid-Typ umsetzt.

4. Verfahren zum kovalenten Aufpfropfen von Trialkylammoniumsalzen nach Anspruch 1 oder 2, **dadurch gekennzeichnet, daß** man ein zu pfropfendes Substrat in basischem Medium in wäßriger oder organischer Lösung behandelt und dann mit einer Lösung von Trialkylammoniumsalzen nach Anspruch 1 oder 2 behandelt.

5. Verfahren nach Anspruch 4, **dadurch gekennzeichnet, daß** die Makromoleküle des Substrats labile Wasserstoffatome enthalten.

6. Biozides Substrat, das nach einem Pfropfverfahren gemäß Anspruch 4 erhältlich ist.

7. Verwendung von Trialkylammoniumsalzen nach Anspruch 1 oder 2 zur Herstellung eines durch Kontakt bioziden oder bakteriostatischen Substrats.
